# EUROPEAN PATENT SPECIFICATION

(11) **EP 1 140 811 B1**
(45) Date of publication and mention of the grant of the patent: **02.08.2006**
(21) Application number: 99963584.0
(22) Date of filing: 20.12.1999
(51) Int. Cl.: C07C 317/24, C07D 265/02, A01N 41/06, A01N 43/72

(54) **NOVEL HERBICIDES**
HERBIZIDE
NOUVEAUX HERBICIDES

(30) Priority: 21.12.1998 CH 252198
(43) Date of publication of application: 10.10.2001
(73) Proprietor: Syngenta Participations AG, 4058 Basel (CH)
(72) Inventor: SCHAETZER, Juergen, D-79618 Rheinfelden (DE); DE MESMAEKER, Alain, CH-4447 Kaenerkinden (CH); LEE, Shy-Fuh, Sunnyvale, CA 94086 (US)
(74) Representative: Hölscher, Ingo
(86) International application number: PCT/EP1999/010128
(87) International publication number: WO 2000/037437

(56) References cited:
- EP-A- 0 338 992
- EP-A- 0 394 889
- US-A- 5 336 662
- US-A- 5 565 410
- US-A- 5 700 762
- US-A- 5 801 120

## Description

The present invention relates to novel herbicidally active benzoyl derivatives, to processes for their preparation, to compositions comprising said compounds, and to the use thereof for controlling weeds, in particular in crops of cultivated plants or for inhibiting plant growth.

Benzoyl derivatives with herbicidal activity are described for example in US-A-5.094.685. Now, novel benzoyl derivatives with herbicidal and growth-inhibiting properties have been found.

The objects of the present invention are thus compounds of formula I 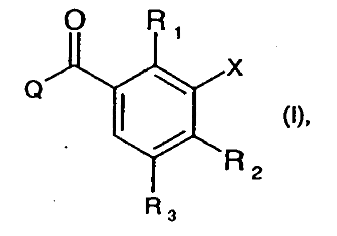 wherein
X is L₁-Y₁-R₄
L₁ signifies C₁-C₆-alkylene
Y₁ signify oxygen, or sulphur
R₁ signifies halogen or C₁-C₆-alkyl; signify halogen, C₁-C₆-halogenalkyl, C₁-C₆-alkylthio, C₁-C₆-alkylsulphinyl, C₁-C₆₋alkylsulphonyl,
R₃ signifies hydrogen, C₁-C₄-alkyl or halogen;
R₄ signifies hydrogen, C₁-C₆-alkyl, which may be substituted by the group A₁;
A₁ is C₁-C₆-alkoxy,
Q is the group Q₁ 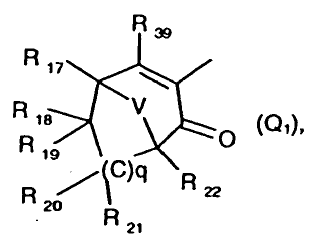
wherein R₃₉ signifies hydroxy
V is methylene, ethylene or oxygen,
R₁₇, R₁₈, R₁₉, R₂₀, R₂₁ and R₂₂, independently of one another, signify hydrogen, or methyl
q is 1 or 2;
or Q is the group Q₂ 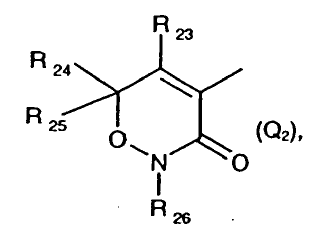
wherein
R₂₃ signifies hydroxy
R₂₄ and R₂₅, independently of one another, signify C₁-C₆-alkyl or R₂₄ and R₂₅ together form a C₂-C₆-alkylene bridge,
R₂₆ signifies hydrogen, C₁-C₄-alkyl, C₁-C₄-alkoxycarbonyl
or Q is the group Q₃ 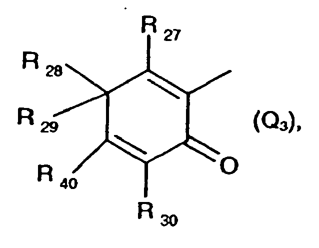
wherein
R₂₇ signifies hydroxy
R₂₈, R₂₉ and R₄₀, independently of one another, signify hydrogen, or methyl;
R₃₀ signifies methyl
or Q is the group Q₄ 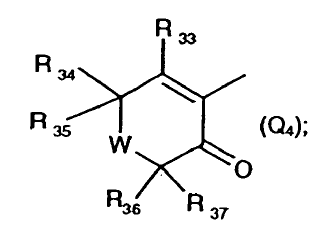
wherein
R₃₃ signifies hydroxy,
W is oxygen or C=O;
R₃₄, R₃₅, R₃₆ and R₃₇, independently of one another, signify hydrogen, or methyl as well as agronomically acceptable salts, isomers and enantiomers of these compounds.

The invention similarly relates to the salts that may be formed by the compounds of formula I, with amines, alkali metal bases and alkaline earth metal bases, or quaternary ammonium bases.

Of the alkali metal hydroxides and alkaline earth metal hydroxides as salt-forming components, the hydroxides of lithium, sodium, potassium, magnesium or calcium are notable, especially those of sodium or potassium.

Examples of amines that are suitable for ammonium salt formation may be both ammonia and primary, secondary and tertiary C₁-C₁₈-alkylamines, C₁-C₄-hydroxyalkylamines and C₂-C₄-alkoxyalkylamines, for example methylamine, ethylamine, n-propylamine, iso-propylamine, the four isomeric butylamines, n-amylamine, iso-amylamine, hexylamine, heptylamine, octylamine, nonylamine, decylamine, pentadecylamine, hexadecylamine, heptadecylamine, octadecylamine, methylethylamine, methyl-iso-propylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di-n-propylamine, di-iso-propylamine, di-n-butylamine, di-n-amylamine, di-iso-amylamine, dihexylamine, diheptylamine, dioctylamine, ethanolamine, n-propanolamine, iso-propanolamine, N,N-diethanolamine, N-ethylpropanolamine, N-butylethanolamine, allylamine, n-butenyl-2-amine, n-pentenyl-2-amine, 2,3-dimethylbutenyl-2-amine, di-butenyl-2-amine, n-hexenyl-2-amine, propylenediamine, trimethylamine, triethylamine, tri-n-propylamine, tri-iso-propylamine, tri-n-butylamine, tri-iso-butylamine, tri-sec.-butylamine, tri-n-amylamine, methoxyethylamine and ethoxyethylamine; heterocyclic amines such as pyridine, quinoline, iso-quinoline, morpholine, piperidine, pyrrolidine, indoline, quinuclidine and azepine; primary arylamines such as anilines, methoxyanilines, ethoxyanilines, o,m,p-toluidines, phenylenediamines, benzidines, naphthylamines and o,m,p-chloroanilines; but especially triethylamine, iso-propylamine and di-iso-propylamine.

The alkyl and alkylene groups present in the definitions of the substituents may be straight-chained or branched and are, for example, methyl, ethyl, n-propyl, iso-propyl, n-butyl, sec.-butyl, iso-butyl, tert.-butyl, pentyl and hexyl, as well as the branched isomers thereof. Alkoxy groups are derived from the said alkyl groups.

Halogen normally signifies fluorine, chlorine, bromine or iodine. The same applies also to halogen in conjunction with other definitions such as halogenalkyl or halogenphenyl. Halogenalkyl groups preferably have a chain length of 1 to 6 carbon atoms. Halogenalkyl is for example fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl; preferably trichloromethyl, difluorochloromethyl, difluoromethyl, trifluoromethyl and dichlorofluoromethyl.

Alkoxy groups preferably have a chain length of 1 to 6 carbon atoms. Alkoxy is for example methoxy, ethoxy, propoxy, i-propoxy, n-butoxy, iso-butoxy, sec.-butoxy and tert.-butoxy as well as the isomers pentyloxy and hexyloxy; preferably methoxy and ethoxy. Alkoxycarbonyl signifies for example methoxycarbonyl, ethoxycarbonyl, propoxycarbonyl, iso-propoxycarbonyl, n-butoxycarbonyl, iso-butoxycarbonyl, sec.-butoxycarbonyl or tert.-butoxycarbonyl; preferably methoxycarbonyl or ethoxycarbonyl. Alkylthio groups preferably have a chain length of 1 to 6 carbon atoms. Alkylthio is for example methylthio, ethylthio, propylthio, iso-propylthio, n-butylthio, iso-butylthio, sec.-butylthio or tert.-butylthio, preferably methylthio and ethylthio. Alkylsulphinyl is for example methylsulphinyl, ethylsulphinyl, propylsulphinyl, iso-propylsulphinyl, n-butylsulphinyl, iso-butylsulphinyl, sec.-butylsulphinyl, tert.-butylsulphinyl; preferably methylsulphinyl and ethylsulphinyl.

Alkylsulphonyl is for example methylsulphonyl, ethylsulphonyl, propylsulphonyl, isopropylsulphonyl, n-butylsulphonyl, iso-butylsulphonyl, sec.-butylsulphonyl or tert.-butylsulphonyl; preferably methylsulphonyl or ethylsulphonyl Alkoxyalkoxy groups preferably have a chain length of 2 to 4 carbon atoms.

Preference is given to compounds of formula I, in which R₁ and R₂, independently of one another, signify halogen, or C₁-C₆-alkyl, and
R₃₃ signifies hydroxy.

Further preferred compounds of formula I are characterised in that Q is Q₁ or Q₂, whereby in the group Q₂, R₂₄, R₂₅ and R₂₆ are preferably hydrogen or C₁-C₆-alkyl. Compounds in which L₁ signifies methylene are preferred. In a further preferred group of compounds of formula I, R₂ signifies C₁-C₆-alkylsulphonyl. Also of interest are compounds of formula I, in which R₁ signifies methyl.

Particularly preferred individual compounds falling within the scope of formula I are: 4-hydroxy-3-(4-methylsulphonyl-3-methoxymethyl-2-methyl-benzoyl)-bicyclo[3.2.1]oct-3-en-2-one and 5-hydroxy-4-(4-methylsulphonyl-3-methoxymethyl-2-methyl-benzoyl)-2,6,6-trimethyl-6.H.-[1,2]oxazin-3-one.

The compounds of formula I may be produced by known processes which are described, for example, in US-A-5,565,410, US-A-5,608,101 and EP-A-0 282 944, whereby e.g. a compound of formula II 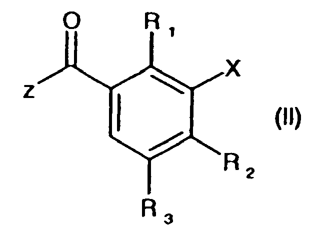 wherein R₁, R₂, R₃ and X have the significances given under formula I and Z signifies a leaving group, preferably halogen, especially chlorine or cyano, is reacted with a compound of formula III 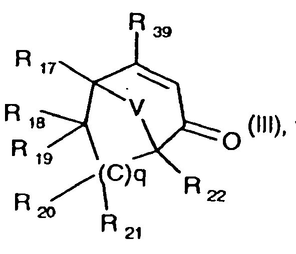 wherein the substituents are defined as in group Q₁, or with a compound of formula IV 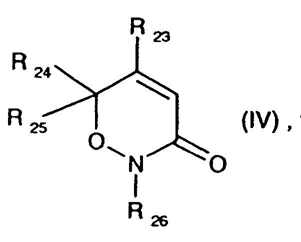 wherein the substituents are defined as in group Q₂, or with a compound of formula V 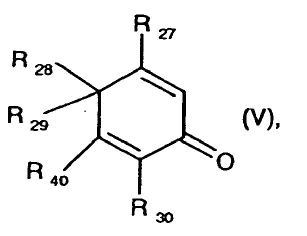 wherein the substituents are defined as in group Q₃, or with a compound of formula VI 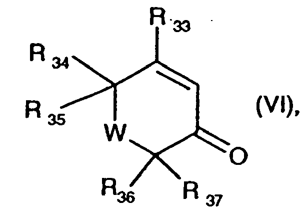 wherein the substituents are defined as in group Q₄ and
correspondingly R₃₉, R₂₃, A₂₇ and A₃₃ signify hydroxy, optionally in the presence of a base. The compounds of formulae II, III, IV or V are known from US-A-5,565,410, US-A-5,608,101 and EP-A-0 282 944 or may be produced analogously to the processes disclosed therein.

The reactions for obtaining the compounds of formula I are advantageously carried out in aprotic inert organic solvents. Such solvents are hydrocarbons such as benzene, toluene, xylene or cyclohexane, chlorinated hydrocarbons such as dichloromethane, trichloromethane, tetrachloromethane or chlorobenzene, ethers such as diethyl ether, ethylene glycol dimethyl ether, diethylene glycol dimethyl ether, tetrahydrofuran or dioxane, nitriles such as acetonitrile or propionitrile, amides such as N,N-dimethyl formamide; diethyl formamide or N-methylpyrrolidinone. The reaction temperatures are preferably in the range from -20° to +120°C. The reactions are usually slightly exothermic and can as a rule be carried out at room temperature. The reaction mixture can be heated for a brief time to boiling point to shorten the reaction time or also to initiate the reaction. The reaction times can also be shortened by addition of a few drops of a base as reaction catalyst. Particularly suitable bases are tertiary amines such as trimethylamine, triethylamine, quinuclidine, 1,4-diazabicyclo[2.2.2]octane, 1,5-diazabicyclo[4.3.0]non-5-ene or 1,5-diazabicyclo[5.4.0]undec-7-ene. Further suitable bases are also inorganic bases, typically hydrides such as sodium or calcium hydride, hydroxides such as sodium or potassium hydroxide, carbonates such as sodium and potassium carbonate, or hydrogen carbonates such as potassium and sodium hydrogen carbonate.
The compounds of formula I can be isolated in conventional manner by concentrating the reaction mixture and/or removing the solvent by evaporation and by recrystallising or triturating the solid residue in solvents in which they are not readily soluble, typically ethers, aromatic hydrocarbons or chlorinated hydrocarbons.

The compounds of formula I or compositions containing them may be used according to this invention by all standard methods of application used in agriculture, including preemergence application, postemergence application and seed dressing, as well as by different methods and techniques such as controlled release. For controlled release, a solution of the herbicide is applied to a mineral granular carrier or to a polymerised granulate (urea/formaldehyde) and then dried. A coating can then be additionally applied (coated granules) that allows the active ingredient to be released at a controlled rate over a specific period of time.

The compounds of formula I may be used as herbicides in unmodified form, i.e. as obtained in the synthesis. Preferably they are processed in conventional manner with the auxiliary agents customarily employed in formulation technology to emulsifiable concentrates, directly sprayable or dilutable solutions, dilute emulsions, wettable powders, soluble powders, dusts, granulates or microcapsules. Such formulations are described, for example, in WO 97/34485 on pages 9 to 13. As with the type of agents, the methods of application such as spraying, atomising, dusting, wetting, scattering or pouring, are selected in accordance with the intended objectives and the prevailing circumstances.

The formulations, i.e. the agents, preparations, or compositions containing the compound of formula I or at least one compound of formula I and usually one or more than one liquid or solid formulation assistant, are prepared in known manner, e.g. by homogeneously mixing and/or grinding the herbicide with said formulation auxiliaries, typically solvents or solid carriers. Surface-active compounds (surfactants) may additionally be used for preparing the formulations. Examples of solvents and solid carriers are described in WO 97/34485 on page 6.

Depending on the herbicide of formula I to be formulated, suitable surface-active compounds are nonionic, cationic and/or anionic surfactants and surfactant mixtures having good emulsifying, dispersing and wetting properties.

Examples of suitable anionic, nonionic, and cationic surfactants are listed for example in WO 97/34485 on pages 7 and 8.

Also the surfactants customary in the art of formulation and described, *inter alia,* in "McCutcheon's Detergents and Emulsifiers Annual" MC Publishing Corp., Ridgewood New Jersey, 1981, Stache, H., "Tensid-Taschenbuch" (Handbook of Surfactants), Carl Hanser Verlag, Munich/Vienna, 1981, and M. and J. Ash, "Encyclopedia of Surfactants", Vol I-III, Chemical Publishing Co., New York, 1980-81, are suitable for manufacture of the herbicides according to the invention.

The herbicidal compositions will as a rule contain from 0.1 to 99 % by weight, preferably from 0.1 to 95% by weight, of herbicide, from 1 to 99.9% by weight, preferably from 5 to 99.8% by weight, of a solid or liquid adjuvant, and from 0 to 25% by weight, preferably from 0.1 to 25% by weight, of a surfactant. Whereas it is preferred to formulate commercial products as concentrates, the end user will normally use dilute formulations. The compositions may also contain further ingredients, such as: stabilisers, e.g. where appropriate epoxidised vegetable oils (epoxidised coconut oil, rapeseed oil, or soybean oil); anti-foaming agents, typically silicone oil; preservatives; viscosity regulators; binders; and tackifiers; as well as fertilisers or other chemical agents.

The compounds of formula I are usually applied with success to the plants or the locus thereof in concentrations of 0.001 to 4 kg/ha, preferably 0.005 to 2 kg/ha. The concentration required to achieve the desired action can be determined by experimentation. It will depend on the type of action, the development stage of the cultivated plant and of the weed, as well as on the application (locus, time, method), and as a resulty of these variables can vary over a wide range.

The compounds of formula I have excellent herbicidal and growth inhibiting properties, which make them suitable for application in crops of cultivated plants, especially in cereals, cotton, soybeans, sugar beet, sugar cane, plantations, rape, maize, and rice; and for the non-selective control of weeds. Crops will also be understood to mean those crops that have been made tolerant to herbicides or classes of herbicides by conventional breeding or genetic engineering methods. The weeds to be controlled may be monocot as well as dicot weeds, typically *Stellaria, Nasturtium, Agrostis, Digitaria, Avena, Setaria, Sinapis, Lolium, Solanum, Echinochloa, Scirpus, Monochoria, Sagittaria, Bromus, Alopecurus, Sorghum halepense, Rottboellia, Cyperus, Abutilon, Sida, Xanthium, Amaranthus, Chenopodium, Ipomoea, Chrysanthemum, Galium, Viola,* and *Veronica.*

The invention is illustrated by the following non-limitative Examples.

### Preparation Examples:

### Example P1: Preparation of 4-methylsulphonyl-3-methoxymethyl-2-methylbenzoic acid chloride:

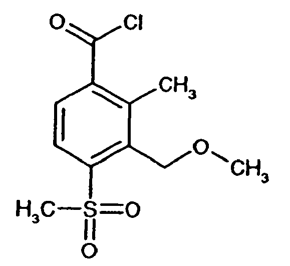

A solution of 1.5 g (5.8 mmols) of 4-methylsulphonyl-3-methoxymethyl-2-methylbenzoic acid in 15 ml of methylene chloride produced in accordance with EP-A-0 282 944 is mixed at a temperature of 20°C with 2 drops of DMF. Then, whilst cooling lightly, a solution of 1.0 ml (11.6 mmols) of oxalyl chloride in 2 ml of methylene chloride is added dropwise. The reaction mixture is stirred until the evolution of gas has ended. Subsequently, the solvent is distilled off. The 4-methylsulphonyl-3-methoxymethyl-2-methylbenzoic acid chloride is used without further purification directly for the next step of the process.

### Example P2: Preparation of 4-hydroxy-3-(4-methylsulphonyl-3-methoxymethyl-2-methylbenzoyl)-bicyclo[3.2.1]oct-3-en-2-one:

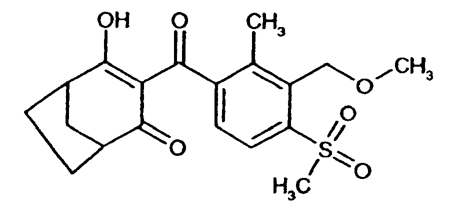

0.8 g (5.8 mmols) of bicyclo[3.2.1]octan-2,4-dione (the preparation thereof is described for example in US-A-5,608,101 and in the references cited therein) are dissolved in 15 ml of methylene chloride at 20°C. The solution is mixed with 0.97 ml of triethylamine and cooled to a temperature of 0°C. Then, a solution of 1.6 g (5.8 mmols) of 4-methylsulphonyl-3-methoxymethyl-2-methylbenzoic acid chloride in 10 ml of methylene chloride is added dropwise. The reaction mixture is stirred for half an hour at a temperature of 0°C and is subsequently diluted with methylene chloride. After washing and drying, the organic phase is concentrated by evaporation. 2.23 g of 4-methylsulphonyl-3-methoxymethyl-2-methylbenzoic acid-4-oxo-bicyclo[3.2.1]oct-2-en-2-yl-ester is obtained in amorphous form. This can be used for the next step without purification.

2.23 g (5.8 mmols) of 4-methylsulphonyl-3-methoxymethyl-2-methyl-benzoic acid-4-oxo-bicyclo[3.2.1]oct-2-en-2-yl-ester and 1.6 ml (11.6 mmols) of triethylamine are dissolved in 20 ml of acetonitrile. 0.2 ml of acetocyanohydrin are added at a temperature of 20°C. After stirring for 20 hours, the mixture is worked up and the crude product is purified by thick-layer chromatography. 1.0 g of 4-hydroxy-3-(4-methylsulphonyl-3-methoxymethyl-2-methylbenzoyl)-bicyclo[3.2.1]oct-3-en-2-one is obtained in amorphous form.

### Example P3: Preparation of 5-hydroxy-4-(4-methylsulphonyl-3-methoxymethyl-2-methylbenzoyl)-2,6,6-trimethyl-6,H.-[1,2]oxazin-3-one:

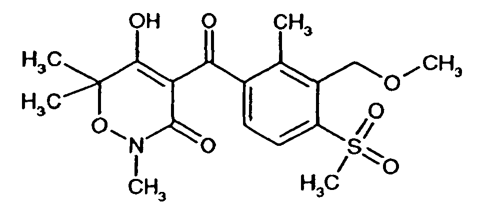

0.79 g (5.04 mmols) of 2,6,6-trimethyl-2H-1,2-oxazin-3,5-dione (the preparation thereof is described for example in US-A-5.565.410), 15 ml of methylene chloride and 0.97 ml of triethylamine are prepared, and at a temperature of 0°C, a solution of 1.3 g (4.7 mmols) of 4-methylsulphonyl-3-methoxymethyl-2-methylbenzoic acid chloride in 10 ml methylene chloride is added dropwise. After stirring for 30 minutes, the solution is diluted with methylene chloride. The reaction solution is subsequently acidified with diluted hydrochloric acid, the organic phase separated, dried and concentrated. 2.0 g of 4-methylsulphonyl-3-methoxymethyl-2-methyl-benzoic acid-2,6,6-trimethyl-5-oxo-5,6-dihydro-2.H-[1,2]oxazin-3-yl-ester are obtained in amorphous form. The product may be further used directly without purification.

2.0 g (5.04 mmols) of 4-methylsulphonyl-3-methoxymethyl-2-methyl-benzoic acid-2,6,6-trimethyl-5-oxo-5,6-dihydro-2.H.-[1,2]oxazin-3-yl-ester are dissolved in a mixture of 35 ml of acetonitrile and 10 ml of methylene chloride, then 1.4 ml (10.08 mmols) of triethylamine and 0.18 ml of acetocyanohydrin are added. After stirring for 20 hours at a temperature of 20°C and then working up, recrystallisation is effected, and finally hydroxy-4-(4-methylsulphonyl-3-methoxymethyl-2-methyl-benzoyl)-2,6,6-trimethyl-6.H.-[1,2]oxazin-3-one is obtained with a melting point of 157°C.

The substances named in the following Tables 1 to 4 may also be prepared analogously to the methods described above. In Tables 1 to 4, X denotes the following groups:
X₁ = CH₂OCH₃, X₂ = CH₂OC₂H₅, X₃ = CH₂OH, X₄ = CH₂CH₂OCH₃, X₅ = CH₂CH₂OC₂H₅, X₆ = CH₂CH₂OCH₂CH₂OCH₃, X₇ = CH₂CH₂OCH₂CH₂OC₂H₅, X₈ = CH(CH₃)OC₂H₅, X₁₁ = CH₂OCH₂CH₂CH₃, X₁₂ = CH(C₂H₅)OC₂H₅, X₁₃ = C(CH₃)₂OH, X₁₄ = CH₂CH₂OCH(CH₃)₂, X₁₈ = CH₂OCH(CH₃)₂, X₁₉ = C(CH₃)₂OCH₃, X₂₀ = CH₂CH₂OH, X₂₂ = C(CH₃)₂OC₂H₅, X₂₃ = CH(C₂H₅)OCH₃, X₂₄ = CH(CH₃)OH, X₂₅ = CH(OH)C₂H₅, X₂₆ = CH₂SCH₃, X₃₂ = CH₂OCH₂CH₂OCH₃, X₃₃ = CH₂OCH₂CH₂OC₂H₅,

**Table 1: Compounds of formula (Ia):**

| | | | | | | | |
|---|---|---|---|---|---|---|---|
| 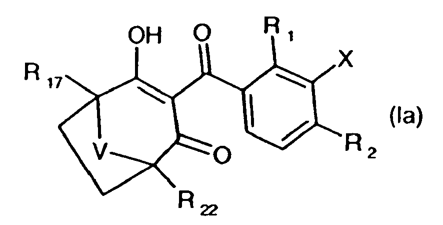 | | | | | | | |

| Comp. No. | V | R₁₇ | R₂₂ | R₁ | X | R₂ | Phys. data |
|---|---|---|---|---|---|---|---|
| 1,001 | CH₂ | H | H | CH₃ | X₁ | SO₂CH₃ | resin |
| 1,002 | CH₂ | H | H | CH₃ | X₂ | SO₂CH₃ | - |
| 1,003 | CH₂ | H | H | CH₃ | X₃ | SO₂CH₃ | - |
| 1,004 | CH₂ | H | H | CH₃ | X₄ | SO₂CH₃ | - |
| 1,005 | CH₂ | H | H | CH₃ | X₅ | SO₂CH₃ | - |
| 1,006 | CH₂ | H | H | CH₃ | X₆ | SO₂CH₃ | - |
| 1,007 | CH₂ | H | H | CH₃ | X₇ | SO₂CH₃ | - |
| 1,008 | CH₂ | H | H | CH₃ | X₈ | SO₂CH₃ | - |
| 1,009 | CH₂ | H | H | CH₃ | X₉ | SO₂CH₃ | - |
| 1,010 | CH₂ | H | H | CH₃ | X₁₁ | SO₂CH₃ | - |
| 1,011 | CH₂ | H | H | CH₃ | X₁₂ | SO₂CH₃ | - |
| 1,012 | CH₂ | H | H | CH₃ | X₁₃ | SO₂CH₃ | - |
| 1,013 | CH₂ | H | H | CH₃ | X₁₄ | SO₂CH₃ | - |
| 1,014 | CH₂ | H | H | CH₃ | X₁₈ | SO₂CH₃ | - |
| 1,015 | CH₂ | H | H | CH₃ | X₁₉ | SO₂CH₃ | - |
| 1,016 | CH₂ | H | H | CH₃ | X₂₀ | SO₂CH₃ | - |
| 1,017 | CH₂ | H | H | CH₃ | X₂₂ | SO₂CH₃ | - |
| 1,018 | CH₂ | H | H | CH₃ | X₂₃ | SO₂CH₃ | - |
| 1,019 | CH₂ | H | H | CH₃ | X₂₄ | SO₂CH₃ | - |
| 1,020 | CH₂ | H | H | CH₃ | X₂₅ | SO₂CH₃ | - |
| 1,021 | CH₂ | H | H | CH₃ | X₂₆ | SO₂CH₃ | - |
| 1,022 | CH₂ | H | H | CH₃ | X₃₂ | SO₂CH₃ | - |
| 1,023 | CH₂ | H | H | CH₃ | X₃₃ | SO₂CH₃ | - |
| 1,024 | CH₂ | H | H | CH₃ | X₁ | SCH₃ | - |
| 1,025 | CH₂ | H | H | CH₃ | X₂ | SCH₃ | - |
| 1,026 | CH₂ | H | H | CH₃ | X₃ | SCH₃ | - |
| 1,027 | CH₂ | H | H | CH₃ | X₄ | SCH₃ | - |
| 1,028 | CH₂ | H | H | CH₃ | X₅ | SCH₃ | - |
| 1,029 | CH₂ | H | H | CH₃ | X₆ | SCH₃ | - |
| 1,030 | CH₂ | H | H | CH₃ | X₇ | SCH₃ | - |
| 1,031 | CH₂ | H | H | CH₃ | X₂₀ | SCH₃ | - |
| 1,032 | CH₂ | H | H | CH₃ | X₂₆ | SCH₃ | - |
| 1,033 | CH₃ | CH₃ | CH₃ | CH₃ | X₁ | SO₂CH₃ | - |
| 1,034 | CH₃ | CH₃ | CH₃ | CH₃ | X₂ | SO₂CH₃ | - |
| 1,035 | CH₃ | CH₃ | CH₃ | CH₃ | X₄ | SO₂CH₃ | - |
| 1,036 | CH₃ | CH₃ | CH₃ | CH₃ | X₂₆ | SO₂CH₃ | - |
| 1,037 | O | CH₃ | CH₃ | CH₃ | X₁ | SO₂CH₃ | - |
| 1,038 | O | CH₃ | CH₃ | CH₃ | X₂ | SO₂CH₃ | - |
| 1,039 | O | CH₃ | CH₃ | CH₃ | X₄ | SO₂CH₃ | - |
| 1,040 | O | CH₃ | CH₃ | CH₃ | X₂₆ | SO₂CH₃ | - |
| 1,041 | CH₂ | H | H | Cl | X₁ | SO₂CH₃ | |
| 1,042 | CH₂ | H | H | Cl | X₂ | SO₂CH₃ | |
| 1,043 | CH₂ | H | H | Cl | X₃ | SO₂CH₃ | |
| 1,044 | CH₂ | H | H | Cl | X₄ | SO₂CH₃ | |
| 1,045 | CH₂ | H | H | Cl | X₅ | SO₂CH₃ | |
| 1,046 | CH₂ | H | H | Cl | X₆ | SO₂CH₃ | |
| 1,047 | CH₂ | H | H | Cl | X₇ | SO₂CH₃ | |
| 1,048 | CH₂ | H | H | Cl | X₈ | SO₂CH₃ | |
| 1,049 | CH₂ | H | H | Cl | X₉ | SO₂CH₃ | |
| 1,050 | CH₂ | H | H | Cl | X₁₁ | SO₂CH₃ | |
| 1,051 | CH₂ | H | H | Cl | X₁₂ | SO₂CH₃ | |
| 1,052 | CH₂ | H | H | Cl | X₁₃ | SO₂CH₃ | |
| 1,053 | CH₂ | H | H | Cl | X₁₄ | SO₂CH₃ | |
| 1,054 | CH₂ | H | H | Cl | X₁₈ | SO₂CH₃ | |
| 1,055 | CH₂ | H | H | Cl | X₁₉ | SO₂CH₃ | |
| 1,056 | CH₂ | H | H | Cl | X₂₀ | SO₂CH₃ | |
| 1,057 | CH₂ | H | H | Cl | X₂₂ | SO₂CH₃ | |
| 1,058 | CH₂ | H | H | Cl | X₂₃ | SO₂CH₃ | |
| 1,059 | CH₂ | H | H | Cl | X₂₄ | SO₂CH₃ | |
| 1,060 | CH₂ | H | H | Cl | X₂₅ | SO₂CH₃ | |
| 1,061 | CH₂ | H | H | Cl | X₂₆ | SO₂CH₃ | |
| 1,062 | CH₂ | H | H | Cl | X₃₂ | SO₂CH₃ | |
| 1,063 | CH₂ | H | H | Cl | X₃₃ | SO₂CH₃ | |
| 1,064 | CH₂ | H | H | CH₃ | X₁ | CF₃ | |
| 1,065 | CH₂ | H | H | CH₃ | X₂ | CF₃ | |
| 1,066 | CH₂ | H | H | CH₃ | X₃ | CF₃ | |
| 1,067 | CH₂ | H | H | CH₃ | X₄ | CF₃ | |
| 1,068 | CH₂ | H | H | CH₃ | X₅ | CF₃ | |
| 1,069 | CH₂ | H | H | CH₃ | X₆ | CF₃ | |
| 1,070 | CH₂ | H | H | CH₃ | X₇ | CF₃ | |
| 1,071 | CH₂ | H | H | CH₃ | X₂₀ | CF₃ | |
| 1,072 | CH₂ | H | H | CH₃ | X₂₆ | CF₃ | |
| 1,073 | CH₂ | H | H | CH₃ | X₁ | Br | |
| 1,074 | CH₂ | H | H | CH₃ | X₂ | Br | |
| 1,075 | CH₂ | H | H | CH₃ | X₃ | Br | |
| 1,076 | CH₂ | H | H | CH₃ | X₄ | Br | |
| 1,077 | CH₂ | H | H | CH₃ | X₅ | Br | |
| 1,078 | CH₂ | H | H | CH₃ | X₆ | Br | |
| 1,079 | CH₂ | H | H | CH₃ | X₇ | Br | |
| 1,080 | CH₂ | H | H | CH₃ | X₂₀ | Br | |
| 1,081 | CH₂ | H | H | CH₃ | X₂₆ | Br | |
| 1,084 | CH₂ | H | H | CH₃ | X₁ | Cl | |
| 1,085 | CH₂ | H | H | CH₃ | X₂ | Cl | |
| 1,086 | CH₂ | H | H | CH₃ | X₃ | Cl | |
| 1,087 | CH₂ | H | H | CH₃ | X₄ | Cl | |
| 1,088 | CH₂ | H | H | CH₃ | X₆ | Cl | |
| 1,089 | CH₂ | H | H | CH₃ | X₂₆ | Cl | |
| 1,090 | CH₂ | H | H | CH₃ | X₃₀ | Cl | |

**Table 2: Compounds of formula (lb):**

| | | | | | | | |
|---|---|---|---|---|---|---|---|
| 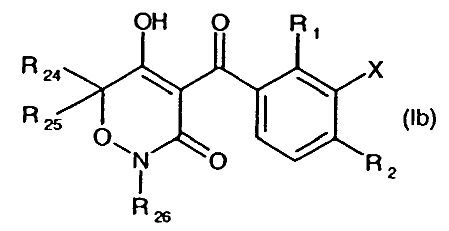 | | | | | | | |

| Comp. No. | R₂₄ | R₂₅ R₂₆ R₁ X R₂ | | | | | m.p. (°C) |
|---|---|---|---|---|---|---|---|
| 2,001 | CH₃ | CH₃ | CH₃ | CH₃ | X₁ | SO₂CH₃ | 157°C |
| 2,002 | CH₃ | CH₃ | CH₃ | CH₃ | X₂ | SO₂CH₃ | - |
| 2,003 | CH₃ | CH₃ | CH₃ | CH₃ | X₃ | SO₂CH₃ | - |
| 2,004 | CH₃ | CH₃ | CH₃ | CH₃ | X₄ | SO₂CH₃ | - |
| 2,005 | CH₃ | CH₃ | CH₃ | CH₃ | X₅ | SO₂CH₃ | - |
| 2,006 | CH₃ | CH₃ | CH₃ | CH₃ | X₆ | SO₂CH₃ | - |
| 2,007 | CH₃ | CH₃ | CH₃ | CH₃ | X₇ | SO₂CH₃ | - |
| 2,008 | CH₃ | CH₃ | CH₃ | CH₃ | X₈ | SO₂CH₃ | - |
| 2,009 | CH₃ | CH₃ | CH₃ | CH₃ | X₉ | SO₂CH₃ | - |
| 2,010 | CH₃ | CH₃ | CH₃ | CH₃ | X₁₁ | SO₂CH₃ | - |
| 2,011 | CH₃ | CH₃ | CH₃ | CH₃ | X₁₂ | SO₂CH₃ | - |
| 2,012 | CH₃ | CH₃ | CH₃ | CH₃ | X₁₃ | SO₂CH₃ | - |
| 2,013 | CH₃ | CH₃ | CH₃ | CH₃ | X₁₄ | SO₂CH₃ | - |
| 2,014 | CH₃ | CH₃ | CH₃ | CH₃ | X₁₈ | SO₂CH₃ | - |
| 2,015 | CH₃ | CH₃ | CH₃ | CH₃ | X₁₉ | SO₂CH₃ | - |
| 2,016 | CH₃ | CH₃ | CH₃ | CH₃ | X₂₀ | SO₂CH₃ | - |
| 2,017 | CH₃ | CH₃ | CH₃ | CH₃ | X₂₂ | SO₂CH₃ | - |
| 2,018 | CH₃ | CH₃ | CH₃ | CH₃ | X₂₃ | SO₂CH₃ | - |
| 2,019 | CH₃ | CH₃ | CH₃ | CH₃ | X₂₄ | SO₂CH₃ | - |
| 2,020 | CH₃ | CH₃ | CH₃ | CH₃ | X₂₅ | SO₂CH₃ | - |
| 2,021 | CH₃ | CH₃ | CH₃ | CH₃ | X₂₆ | SO₂CH₃ | - |
| 2,022 | CH₃ | CH₃ | CH₃ | CH₃ | X₃₂ | SO₂CH₃ | - |
| 2,023 | CH₃ | CH₃ | CH₃ | CH₃ | X₃₃ | SO₂CH₃ | - |
| 2,024 | CH₃ | CH₃ | CH₃ | Cl | X₁ | SO₂CH₃ | - |
| 2,025 | CH₃ | CH₃ | CH₃ | Cl | X₂ | SO₂CH₃ | - |
| 2,026 | CH₃ | CH₃ | CH₃ | Cl | X₃ | SO₂CH₃ | - |
| 2,027 | CH₃ | CH₃ | CH₃ | Cl | X₄ | SO₂CH₃ | - |
| 2,028 | CH₃ | CH₃ | CH₃ | Cl | X₅ | SO₂CH₃ | - |
| 2,029 | CH₃ | CH₃ | CH₃ | Cl | X₆ | SO₂CH₃ | - |
| 2,030 | CH₃ | CH₃ | CH₃ | Cl | X₇ | SO₂CH₃ | - |
| 2,031 | CH₃ | CH₃ | CH₃ | Cl | X₂₀ | SO₂CH₃ | - |
| 2,032 | CH₃ | CH₃ | CH₃ | Cl | X₂₆ | SO₂CH₃ | - |
| 2,033 | CH₃ | CH₃ | CH₃ | CH₃ | X₁ | SCH₃ | - |
| 2,034 | CH₃ | CH₃ | CH₃ | CH₃ | X₂ | SCH₃ | - |
| 2,035 | CH₃ | CH₃ | CH₃ | CH₃ | X₃ | SCH₃ | - |
| 2,036 | CH₃, | CH₃ | CH₃ | CH₃ | X₄ | SCH₃ | - |
| 2,037 | CH₃ | CH₃ | CH₃ | CH₃ | X₅ | SCH₃ | - |
| 2,038 | CH₃ | CH₃ | CH₃ | CH₃ | X₆ | SCH₃ | - |
| 2,039 | CH₃ | CH₃ | CH₃ | CH₃ | X₇ | SCH₃ | - |
| 2,040 | CH₃ | CH₃ | CH₃ | CH₃ | X₂₀ | SCH₃ | - |
| 2,041 | CH₃ | CH₃ | CH₃ | CH₃ | X₂₆ | SCH₃ | - |
| 2,042 | CH₃ | CH₃ | CH₃ | Cl | X₁ | SCH₃ | - |
| 2,043 | CH₃ | CH₃ | CH₃ | Cl | X₂ | SCH₃ | - |
| 2,044 | CH₃ | CH₃ | CH₃ | Cl | X₄ | SCH₃ | - |
| 2,045 | CH₃ | CH₃ | CH₃ | Cl | X₂₆ | SCH₃ | - |
| 2,046 | CH₃ | CH₃ | CH₃ | CH₃ | X₁ | CF₃ | - |
| 2,047 | CH₃ | CH₃ | CH₃ | CH₃ | X₂ | CF₃ | - |
| 2,048 | CH₃ | CH₃ | CH₃ | CH₃ | X₃ | CF₃ | - |
| 2,049 | CH₃ | CH₃ | CH₃ | CH₃ | X₄ | CF₃ | - |
| 2,050 | CH₃ | CH₃ | CH₃ | CH₃ | X₆ | CF₃ | - |
| 2,051 | CH₃ | CH₃ | CH₃ | CH₃ | X₂₆ | CF₃ | - |
| 2,052 | CH₃ | CH₃ | CH₃ | Br | X₁ | SO₂CH₃ | - |
| 2,053 | CH₃ | CH₃ | CH₃ | Br | X₂ | SO₂CH₃ | - |
| 2,054 | CH₃ | CH₃ | CH₃ | Br | X₄ | SO₂CH₃ | - |
| 2,055 | CH₃ | CH₃ | CH₃ | Br | X₂₆ | SO₂CH₃ | - |

**Table 3: Compounds of formula (Ic):**

| | | | | | | | |
|---|---|---|---|---|---|---|---|
| 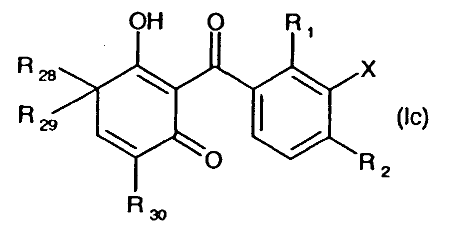 | | | | | | | |

| Comp. No. | R₂₈ | R₂₉ | R₃₀ | R₁ | X | R₂ | Phys. data |
|---|---|---|---|---|---|---|---|
| 3,001 | CH₃ | CH₃ | CH₃ | CH₃ | X₁ | SO₂CH₃ | - |
| 3,002 | CH₃ | CH₃ | CH₃ | CH₃ | X₂ | SO₂CH₃ | - |
| 3,003 | CH₃ | CH₃ | CH₃ | CH₃ | X₃ | SO₂CH₃ | - |
| 3,004 | CH₃ | CH₃ | CH₃ | CH₃ | X₄ | SO₂CH₃ | - |
| 3,005 | CH₃ | CH₃ | CH₃ | CH₃ | X₅ | SO₂CH₃ | - |
| 3,006 | CH₃ | CH₃ | CH₃ | CH₃ | X₆ | SO₂CH₃ | - |
| 3,007 | CH₃ | CH₃ | CH₃ | CH₃ | X₇ | SO₂CH₃ | - |
| 3,008 | CH₃ | CH₃ | CH₃ | CH₃ | X₈ | SO₂CH₃ | - |
| 3,009 | CH₃ | CH₃ | CH₃ | CH₃ | X₉ | SO₂CH₃ SO₂CH₃ | - |
| 3,010 | CH₃ | CH₃ | CH₃ | CH₃ | X₁₁ | SO₂CH₃ | - |
| 3,011 | CH₃ | CH₃ | CH₃ | CH₃ | X₁₂ | SO₂CH₃ | - |
| 3,012 | CH₃ | CH₃ | CH₃ | CH₃ | X₁₃ | SO₂CH₃ | - |
| 3,013 | CH₃ | CH₃ | CH₃ | CH₃ | X₁₄ | SO₂CH₃ | - |
| 3,014 | CH₃ | CH₃ | CH₃ | CH₃ | X₁₈ | SO₂CH₃ | - |
| 3,015 | CH₃ | CH₃ | CH₃ | CH₃ | X₁₉ | SO₂CH₃ | - |
| 3,016 | CH₃ | CH₃ | CH₃ | CH₃ | X₂₀ | SO₂CH₃ | - |
| 3,017 | CH₃ | CH₃ | CH₃ | CH₃ | X₂₂ | SO₂CH₃ | - |
| 3,018 | CH₃ | CH₃ | CH₃ | CH₃ | X₂₃ | SO₂CH₃ | - |
| 3,019 | CH₃ | CH₃ | CH₃ | CH₃ | X₂₄ | SO₂CH₃ | - |
| 3,020 | CH₃ | CH₃ | CH₃ | CH₃ | X₂₅ | SO₂CH₃ | - |
| 3,021 | CH₃ | CH₃ | CH₃ | CH₃ | X₂₆ | SO₂CH₃ | - |
| 3,022 | CH₃ | CH₃ | CH₃ | CH₃ | X₃₂ | SO₂CH₃ | - |
| 3,023 | CH₃ | CH₃ | CH₃ | CH₃ | X₃₃ | SO₂CH₃ | - |
| 3,024 | CH₃ | CH₃ | CH₃ | CH₃ | X₁ | SCH₃ | - |
| 3,025 | CH₃ | CH₃ | CH₃ | CH₃ | X₂ | SCH₃ | - |
| 3,026 | CH₃ | CH₃ | CH₃ | CH₃ | X₃ | SCH₃ | - |
| 3,027 | CH₃ | CH₃ | CH₃ | CH₃ | X₄ | SCH₃ | - |
| 3,028 | CH₃ | CH₃ | CH₃ | CH₃ | X₅ | SCH₃ | - |
| 3,029 | CH₃ | CH₃ | CH₃ | CH₃ | X₆ | SCH₃ | - |
| 3,030 | CH₃ | CH₃ | CH₃ | CH₃ | X₇ | SCH₃ | - |
| 3,031 | CH₃ | CH₃ | CH₃ | CH₃ | X₂₀ | SCH₃ | - |
| 3,032 | CH₃ | CH₃ | CH₃ | CH₃ | X₂₆ | SCH₃ | - |
| 3,033 | CH₃ | CH₃ | CH₃ | Cl | X₁ | SO₂CH₃ | - |
| 3,034 | CH₃ | CH₃ | CH₃ | Cl | X₂ | SO₂CH₃ | - |
| 3,035 | CH₃ | CH₃ | CH₃ | Cl | X₃ | SO₂CH₃ | - |
| 3,036 | CH₃ | CH₃ | CH₃ | Cl | X₄ | SO₂CH₃ | - |
| 3,037 | CH₃ | CH₃ | CH₃ | Cl | X₅ | SO₂CH₃ | - |
| 3,038 | CH₃ | CH₃ | CH₃ | Cl | X₆ | SO₂CH₃ | - |
| 3,039 | CH₃ | CH₃ | CH₃ | Cl | X₇ | SO₂CH₃ | - |
| 3,040 | CH₃ | CH₃ | CH₃ | Cl | X₈ | SO₂CH₃ | - |
| 3,041 | CH₃ | CH₃ | CH₃ | Cl | X₉ | SO₂CH₃ | - |
| 3,042 | CH₃ | CH₃ | CH₃ | Cl | X₁₁ | SO₂CH₃ | - |
| 3,043 | CH₃ | CH₃ | CH₃ | Cl | X₁₂ | SO₂CH₃ | - |
| 3,044 | CH₃ | CH₃ | CH₃ | Cl | X₁₃ | SO₂CH₃ | - |
| 3,045 | CH₃ | CH₃ | CH₃ | Cl | X₁₄ | SO₂CH₃ | - |
| 3,046 | CH₃ | CH₃ | CH₃ | Cl | X₁₈ | SO₂CH₃ | - |
| 3,047 | CH₃ | CH₃ | CH₃ | Cl | X₁₉ | SO₂CH₃ | - |
| 3,048 | CH₃ | CH₃ | CH₃ | Cl | X₂₀ | SO₂CH₃ | - |
| 3,049 | CH₃ | CH₃ | CH₃ | Cl | X₂₂ | SO₂CH₃ | - |
| 3,050 | CH₃ | CH₃ | CH₃ | Cl | X₂₃ | SO₂CH₃ | - |
| 3,051 | CH₃ | CH₃ | CH₃ | Cl | X₂₄ | SO₂CH₃ | - |
| 3,052 | CH₃ | CH₃ | CH₃ | Cl | X₂₅ | SO₂CH₃ | - |
| 3,053 | CH₃ | CH₃ | CH₃ | Cl | X₂₆ | SO₂CH₃ | - |
| 3,054 | CH₃ | CH₃ | CH₃ | Cl | X₃₂ | SO₂CH₃ | - |
| 3,055 | CH₃ | CH₃ | CH₃ | Cl | X₃₃ | SO₂CH₃ | - |
| 3,056 | CH₃ | CH₃ | CH₃ | CH₃ | X₁ | CF₃ | - |
| 3,057 | CH₃ | CH₃ | CH₃ | CH₃ | X₂ | CF₃ | - |
| 3,058 | CH₃ | CH₃ | CH₃ | CH₃ | X₃ | CF₃ | - |
| 3,059 | CH₃ | CH₃ | CH₃ | CH₃ | X₄ | CF₃ | - |
| 3,060 | CH₃ | CH₃ | CH₃ | CH₃ | X₆ | CF₃ | - |
| 3,061 | CH₃ | CH₃ | CH₃ | CH₃ | X₂₆ | CF₃ | - |
| 3,062 | CH₃ | CH₃ | CH₃ | Cl | X₁ | SCH₃ | - |
| 3,063 | CH₃ | CH₃ | CH₃ | Cl | X₂ | SCH₃ | - |
| 3,064 | CH₃ | CH₃ | CH₃ | Cl | X₃ | SCH₃ | - |
| 3,065 | CH₃ | CH₃ | CH₃ | Cl | X₄ | SCH₃ | - |
| 3,066 | CH₃ | CH₃ | CH₃ | Cl | X₅ | SCH₃ | - |
| 3,067 | CH₃ | CH₃ | CH₃ | Cl | X₆ | SCH₃ | - |
| 3,068 | CH₃ | CH₃ | CH₃ | Cl | X₇ | SCH₃ | - |
| 3,069 | CH₃ | CH₃ | CH₃ | Cl | X₂₀ | SCH₃ | - |
| 3,070 | CH₃ | CH₃ | CH₃ | Cl | X₂₆ | SCH₃ | - |

**Table 4: Compounds of formula (Id):**

| | | | | | | | |
|---|---|---|---|---|---|---|---|
| 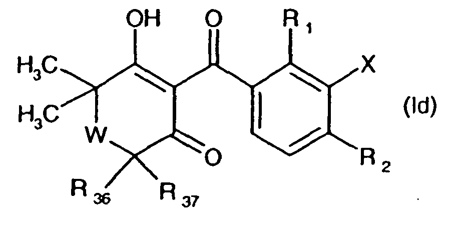 | | | | | | | |

| Comp. No. | R₃₆ | R₃₇ | W | R₁ | X | R₂ | Phys. data |
|---|---|---|---|---|---|---|---|
| 4,001 | CH₃ | CH₃ | O | CH₃ | X₁ | SO₂CH₃ | - |
| 4,002 | CH₃ | CH₃ | O | CH₃ | X₂ | SO₂CH₃ | - |
| 4,003 | CH₃ | CH₃ | O | CH₃ | X₃ | SO₂CH₃ | - |
| 4,004 | CH₃ | CH₃ | O | CH₃ | X₄ | SO₂CH₃ | - |
| 4,005 | CH₃ | CH₃ | O | CH₃ | X₅ | SO₂CH₃ | - |
| 4,006 | CH₃ | CH₃ | O | CH₃ | X₆ | SO₂CH₃ | - |
| 4,007 | CH₃ | CH₃ | O | CH₃ | X₇ | SO₂CH₃ | - |
| 4,008 | CH₃ | CH₃ | O | CH₃ | X₈ | SO₂CH₃ | - |
| 4,009 | CH₃ | CH₃ | O | CH₃ | X₉ | SO₂CH₃ | - |
| 4,010 | CH₃ | CH₃ | O | CH₃ | X₁₁ | SO₂CH₃ | - |
| 4,011 | CH₃ | CH₃ | O | CH₃ | X₁₂ | SO₂CH₃ | - |
| 4,012 | CH₃ | CH₃ | O | CH₃ | X₁₃ | SO₂CH₃ | - |
| 4,013 | CH₃ | CH₃ | O | CH₃ | X₁₄ | SO₂CH₃ | - |
| 4,014 | CH₃ | CH₃ | O | CH₃ | X₁₈ | SO₂CH₃ | - |
| 4,015 | CH₃ | CH₃ | O | CH₃ | X₁₉ | SO₂CH₃ | - |
| 4,016 | CH₃ | CH₃ | O | CH₃ | X₂₀ | SO₂CH₃ | - |
| 4,017 | CH₃ | CH₃ | O | CH₃ | X₂₂ | SO₂CH₃ | - |
| 4,018 | CH₃ | CH₃ | O | CH₃ | X₂₃ | SO₂CH₃ | - |
| 4,019 | CH₃ | CH₃ | O | CH₃ | X₂₄ | SO₂CH₃ | - |
| 4,020 | CH₃ | CH₃ | O | CH₃ | X₂₅ | SO₂CH₃ | - |
| 4,021 | CH₃ | CH₃ | O | CH₃ | X₂₆ | SO₂CH₃ | - |
| 4,022 | CH₃ | CH₃ | O | CH₃ | X₃₂ | SO₂CH₃ | - |
| 4,023 | CH₃ | CH₃ | O | CH₃ | X₃₃ | SO₂CH₃ | - |
| 4,024 | CH₃ | CH₃ | O | CH₃ | X₁ | SCH₃ | - |
| 4,025 | CH₃ | CH₃ | O | CH₃ | X₂ | SCH₃ | - |
| 4,026 | CH₃ | CH₃ | O | CH₃ | X₃ | SCH₃ | - |
| 4,027 | CH₃ | CH₃ | O | CH₃ | X₄ | SCH₃ | - |
| 4,028 | CH₃ | CH₃ | O | CH₃ | X₅ | SCH₃ | - |
| 4,029 | CH₃ | CH₃ | O | CH₃ | X₆ | SCH₃ | - |
| 4,030 | CH₃ | CH₃ | O | CH₃ | X₇ | SCH₃ | - |
| 4,031 | CH₃ | CH₃ | O | CH₃ | X₂₀ | SCH₃ | - |
| 4,032 | CH₃ | CH₃ | O | CH₃ | X₂₆ | SCH₃ | - |
| 4,033 | CH₃ | CH₃ | O | Cl | X₁ | SO₂CH₃ | - |
| 4,034 | CH₃ | CH₃ | O | Cl | X₂ | SO₂CH₃ | - |
| 4,035 | CH₃ | CH₃ | O | Cl | X₃ | SO₂CH₃ | - |
| 4,036 | CH₃ | CH₃ | O | Cl | X₄ | SO₂CH₃ | - |
| 4,037 | CH₃ | CH₃ | O | Cl | X₅ | SO₂CH₃ | - |
| 4,038 | CH₃ | CH₃ | O | Cl | X₆ | SO₂CH₃ | - |
| 4,039 | CH₃ | CH₃ | O | Cl | X₇ | SO₂CH₃ | - |
| 4,040 | CH₃ | CH₃ | O | Cl | X₈ | SO₂CH₃ | - |
| 4,041 | CH₃ | CH₃ | O | Cl | X₉ | SO₂CH₃ | - |
| 4,042 | CH₃ | CH₃ | O | Cl | X₁₁ | SO₂CH₃ | - |
| 4,043 | CH₃ | CH₃ | O | Cl | X₁₂ | SO₂CH₃ | - |
| 4,044 | CH₃ | CH₃ | O | Cl | X₁₃ | SO₂CH₃ | - |
| 4,045 | CH₃ | CH₃ | O | Cl | X₁₄ | SO₂CH₃ | - |
| 4,046 | CH₃ | CH₃ | O | Cl | X₁₈ | SO₂CH₃ | - |
| 4,047 | CH₃ | CH₃ | O | Cl | X₁₉ | SO₂CH₃ | - |
| 4,048 | CH₃ | CH₃ | O | Cl | X₂₀ | SO₂CH₃ | - |
| 4,049 | CH₃ | CH₃ | O | Cl | X₂₂ | SO₂CH₃ | - |
| 4,050 | CH₃ | CH₃ | O | Cl | X₂₃ | SO₂CH₃ | - |
| 4,051 | CH₃ | CH₃ | O | Cl | X₂₄ | SO₂CH₃ | - |
| 4,052 | CH₃ | CH₃ | O | Cl | X₂₅ | SO₂CH₃ | - |
| 4,053 | CH₃ | CH₃ | O | Cl | X₂₆ | SO₂CH₃ | - |
| 4,054 | CH₃ | CH₃ | O | Cl | X₃₂ | SO₂CH₃ | - |
| 4,055 | CH₃ | CH₃ | O | Cl | X₃₃ | SO₂CH₃ | - |
| 4,056 | CH₃ | CH₃ | O | CH₃ | X₁ | CF₃ | - |
| 4,057 | CH₃ | CH₃ | O | CH₃ | X₂ | CF₃ | - |
| 4,058 | CH₃ | CH₃ | O | CH₃ | X₃ | CF₃ | - |
| 4,059 | CH₃ | CH₃ | O | CH₃ | X₄ | CF₃ | - |
| 4,060 | CH₃ | CH₃ | O | CH₃ | X₆ | CF₃ | - |
| 4,061 | CH₃ | CH₃ | O | CH₃ | X₂₆ | CF₃ | - |
| 4,062 | CH₃ | CH₃ | O | NO₂ | X₁ | SO₂CH₃ | - |
| 4,063 | CH₃ | CH₃ | O | NO₂ | X₂ | SO₂CH₃ | - |
| 4,064 | CH₃ | CH₃ | O | NO₂ | X₄ | SO₂CH₃ | - |
| 4,065 | CH₃ | CH₃ | O | NO₂ | X₂₆ | SO₂CH₃ | - |
| 4,066 | CH₃ | CH₃ | O | NO₂ | X₁ | CF₃ | - |
| 4,067 | CH₃ | CH₃ | O | NO₂ | X₂ | CF₃ | - |
| 4,068 | CH₃ | CH₃ | O | NO₂ | X₄ | CF₃ | - |
| 4,069 | CH₃ | CH₃ | O | NO₂ | X₂₆ | CF₃ | - |
| 4,070 | CH₃ | CH₃ | O | Cl | X₁ | SCH₃ | - |
| 4,071 | CH₃ | CH₃ | O | Cl | X₂ | SCH₃ | - |
| 4,072 | CH₃ | CH₃ | O | Cl | X₃ | SCH₃ | - |
| 4,073 | CH₃ | CH₃ | O | Cl | X₄ | SCH₃ | - |
| 4,074 | CH₃ | CH₃ | O | Cl | X₅ | SCH₃ | - |
| 4,075 | CH₃ | CH₃ | O | Cl | X₆ | SCH₃ | - |
| 4,076 | CH₃ | CH₃ | O | Cl | X₇ | SCH₃ | - |
| 4,077 | CH₃ | CH₃ | O | Cl | X₂₀ | SCH₃ | - |
| 4,078 | CH₃ | CH₃ | O | Cl | X₂₆ | SCH₃ | - |

### Biological Examples

### Example B1: Pre-emergent herbicidal action

Monocot and dicot test plants are sown in standard soil in plastic pots. Immediately after sowing, the plants are sprayed at a concentration of 2 kg active substance/ha with an aqueous suspension of the test compound [prepared from a 25% wettable powder (Example F3, b) in accordance with WO 97/34485] or an emulsion of the test compound [prepared from a 25% emulsifiable concentrate (Example F1 c)] (500 I of water/ha). The test plants are then cultivated in the greenhouse under optimum conditions. The test is evaluated 3 weeks later on a rating scale of 1-9 (1 = total damage, 9 = no action). Ratings of 1 to 4 (especially of 1 to 3) denote good to very good herbicidal action.

**Table B1: Pre-emergent action:**

| test plant | Setaria | Cyperus | Sinapsis | Solanig | Stellaria | dosage [g AS/ha] |
|---|---|---|---|---|---|---|
| Active ingredient No. | | | | | | |
| 1,001 | 3 | 3 | 3 | 2 | 2 | 2000 |
| 2,001 | 3 | 3 | 2 | 2 | 2 | 2000 |

The same results are obtained by formulating the compounds of formula I in accordance with Examples F2 and F4 to F8 of WO 97/34485.

### Example B2: Post-emergent herbicidal action

In a greenhouse, monocot and dicot test plants are sown in standard soil in plastic pots and sprayed in the 4- to 6-leaf stage with an aqueous suspension of the test compounds of formula I prepared from a 25 % wettable powder [Example F3, b) of WO 97/34485] or with an emulsion of the test compound of formula I prepared from a 25 % emulsifiable concentrate [Example F1 c) of WO 97/34485] at a concentration of 2 kg active substance/ha (500 I of water/ha). The test plants are then further cultivated in the greenhouse under optimum conditions. The test is evaluated about 18 days later on a rating scale of 1-9 (1 = total damage, 9 = no action). Ratings of 1 to 4 (especially of 1 to 3) denote good to very good herbicidal action. In this test the compounds of formula I exhibit a pronounced herbicidal action.

**Table B2: Post-emergent action**

| test plant | Setaria | Cyperus | Sinapsis | Solanig | Stellaria | dosage [g AS/ha] |
|---|---|---|---|---|---|---|
| Active ingredient No. | | | | | | |
| 1,001 | 2 | 3 | 2 | 2 | 2 | 2000 |
| 2,001 | 3 | 3 | 2 | 2 | 4 | 2000 |

The same results are obtained by formulating the compounds of formula I in accordance with Examples F2 and F4 to F8 of WO 97/34485.

## Claims

1. Compounds of formula I 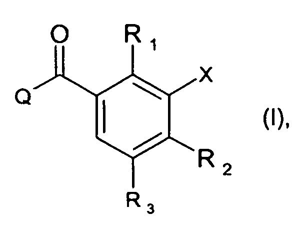 wherein
X is L₁-Y₁-R₄;
L₁ signifies C₁-C₆-alkylene;
Y₁ signifies oxygen or sulphur;
R₁ signifies halogen or C₁-C₆-alkyl;
R₂ signifies halogen C₁-C₆-halogenalkyl, C₁-C₆-alkylthio, C₁-C₆-alkylsulphinyl or C₁-C₆₋alkylsulphonyl;
R₃ signifies hydrogen, C₁-C₄-alkyl or halogen;
R₄ signify hydrogen, C₁-C₆-alkyl, which may be substituted by the group A₁;
A₁ is C₁-C₆-alkoxy;
Q is the group Q₁ 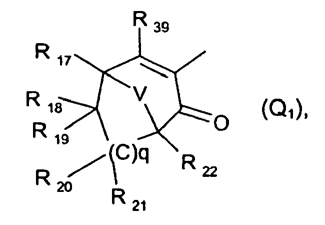
wherein R₃₉ signifies hydroxy; V is methylene, ethylene or oxygen;
R₁₇, R₁₈, R₁₉, R₂₀ , R₂₁, and R₂₂, independently of one another, signify hydrogen or methyl; q is 1 or 2;
or Q is the group Q₂ 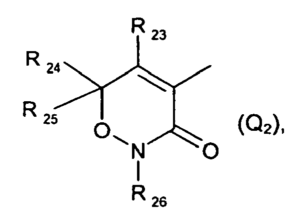
wherein
R₂₃ signifies hydroxy;
R₂₄ and R₂₅, independently of one another, signify C₁-C₆-alkyl, or R₂₄ and R₂₅ together form a C₂-C₆-alkylene bridge,
R₂₆ signifies hydrogen, C₁-C₄-alkyl or C₁-C₄-alkoxycarbonyl;
or Q is the group Q₃ 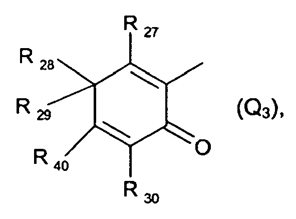
wherein
R₂₇ signifies hydroxy;
R₂₈, R₂₉ and R₄₀, independently of one another, signify hydrogen or methyl;
R₃₀ signifies methyl;
or Q is the group Q₄ 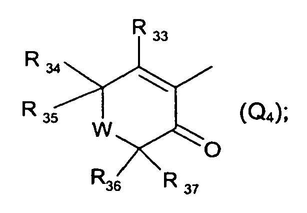
wherein
R₃₃ signifies hydroxy;
W is oxygen or C=O;
R₃₄, R₃₅, R₃₆ and R₃₇, independently of one another, signify hydrogen or methyl, as well as agronomically acceptable salts, isomers and enantiomers of these compounds.

2. Compounds according to claim 1, whereby Q is Q₁ or Q₂.

3. Compounds according to claim 1, whereby L₁ signifies methylene.

4. Compounds according to claim 1, whereby R₂ signifies C₁-C₆-alkylsulphonyl.

5. A herbicidal and plant growth inhibiting composition, which comprises a herbicidally effective amount of the compound of formula I on an inert carrier.

6. A method of controlling undesirable plant growth, which comprises treating the plants or the locus thereof with a herbicidally effective amount of a compound of formula I or of a composition containing such a compound.

7. A method of inhibiting undesirable plant growth, which comprises treating the plants or the locus thereof with a herbicidally effective amount of a compound of formula I or of a composition containing such a compound.

8. Use of a composition according to claim 5 for controlling undesirable plant growth.

## Patentansprüche

1. Verbindungen der Formel I 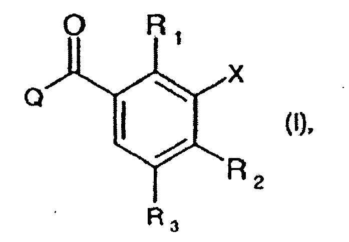 worin
X L₁-Y₁-R₄ darstellt;
L₁ C₁-C₆-Alkylen bedeutet;
Y₁ Sauerstoff oder Schwefel bedeutet;
R₁ Halogen oder C₁-C₆-Alkyl bedeutet;
R₂ Halogen, C₁-C₆-Halogenalkyl, C₁-C₆-Alkylthio, C₁-C₆-Alkylsulfinyl oder C₁-C₆-Alkylsulfonyl bedeutet;
R₃ Wasserstoff, C₁-C₄-Alkyl oder Halogen bedeutet;
R₄ Wasserstoff, C₁-C₆-Alkyl, das mit der Gruppe A₁ substituiert sein kann, bedeutet;
A₁ C₁-C₆-Alkoxy darstellt;
Q die Gruppe Q₁ 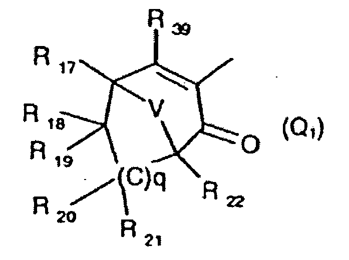
darstellt,
worin
R₃₉ Hydroxy bedeutet; V Methylen, Ethylen oder Sauerstoff darstellt;
R₁₇, R₁₈ R₁₉, R₂₀, R₂₁ und R₂₂ unabhängig voneinander Wasserstoff oder Methyl bedeuten;
q 1 oder 2 ist; oder
Q die Gruppe Q₂ 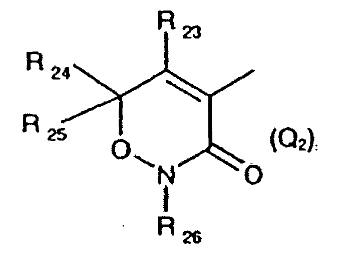
darstellt,
worin
R₂₃ Hydroxy bedeutet;
R₂₄ und R₂₅ unabhängig voneinander C₁-C₆-Alkyl bedeuten oder R₂₄ und R₂₅ zusammen eine C₂-C₆-Alkylenbrücke bilden;
R₂₆ Wasserstoff, C₁-C₄-Alkyl oder C₁-C₄-Alkoxycarbonyl bedeutet; oder
Q die Gruppe Q₃ 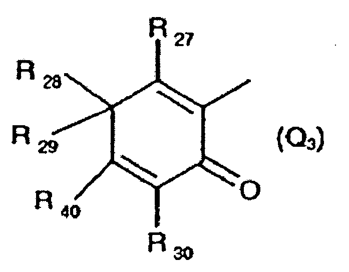
darstellt,
worin
R₂₇ Hydroxy bedeutet;
R₂₈, R₂₉ und R₄₀ unabhängig voneinander Wasserstoff oder Methyl bedeuten;
R₃₀ Methyl bedeutet oder
Q die Gruppe Q₄ 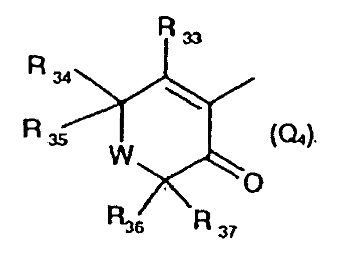
darstellt,
worin
R₃₃ Hydroxy bedeutet;
W Sauerstoff oder C=O darstellt;
R₃₄, R₃₅, R₃₆ und R₃₇ unabhängig voneinander Wasserstoff oder Methyl bedeuten
sowie landwirtschaftlich verträgliche Salze, Isomeren und Enantiomeren von diesen Verbindungen.

2. Verbindungen nach Anspruch 1, wobei Q Q₁ oder Q₂ darstellt.

3. Verbindungen nach Anspruch 1, wobei L₁ Methylen bedeutet.

4. Verbindungen nach Anspruch 1, wobei R₂ C₁-C₆-Alkylsulfonyl bedeutet.

5. Herbizide und pflanzenwuchsinhibierende Zusammensetzung, die eine herbizid wirksame Menge der Verbindung der Formel I auf einem inerten Träger umfasst.

6. Verfahren zum Bekämpfen von unerwünschtem Pflanzenwuchs, das das Behandeln der Pflanzen oder deren Standort mit einer herbizid wirksamen Menge einer Verbindung der Formel I oder von einer eine solche Verbindung enthaltenden Zusammensetzung umfasst.

7. Verfahren zum Hemmen von unerwünschtem Pflanzenwuchs, das das Behandeln der Pflanzen oder deren Standort mit einer herbizid wirksamen Menge einer Verbindung der Formel I oder einer eine solche Verbindung enthaltenden Zusammensetzung umfasst.

8. Verwendung einer Zusammensetzung nach Anspruch 5 zum Bekämpfen von unerwünschtem Pflanzenwuchs.

## Revendications

1. Composés de formule I 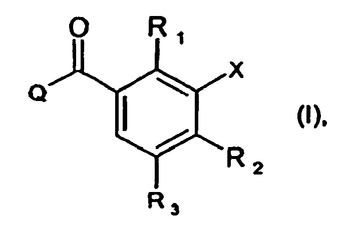 dans laquelle
X est L₁-Y₁-R₄ :
L₁ représente un alkylène en C₁ à C₆ ;
Y₁ représente un oxygène ou soufre ;
R₁ représente un halogène ou alkyle en C₁ à C₆ ;
R₂ représente un halogène, halogénoalkyle en C₁ à C₆, alkylthio en C₁ à C₆, alkylsulfinyle en C₁ à C₆ ou alkylsulfonyle en C₁ à C₆ ;
R₃ représente un hydrogène, alkyle en C₁ à C₄ ou halogène ;
R₄ représente un hydrogène, alkyle en C₁ à C₆, qui peut être substitué par le groupe A₁ ;
A₁ est un alcoxy en C₁ à C₆ ;
Q est le groupe Q₁ 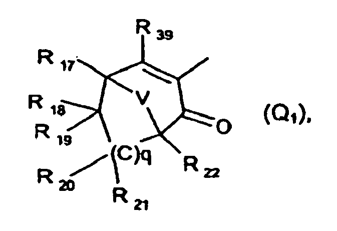
dans lequel R₃₉ représente un hydroxy ; V est un méthylène, éthylène ou oxygène ;
R₁₇, R₁₈, R₁₉, R₂₀, R₂₁ et R₂₂, indépendamment l' un de l'autre, représentent un hydrogène ou méthyle ;
q est 1 ou 2 ;
ou Q est le groupe Q₂ 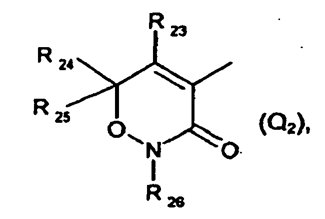 dans lequel
R₂₃ représente un hydroxy ;
R₂₄ et R₂₅, indépendamment l'un de l'autre, représentent un alkyle en C₁ à C₆, ou R₂₄ et R₂₅ forment ensemble un pont alkylène en C₂ à C₆;
R₂₆ représente un hydrogène, alkyle en C₁ à C₄ ou (alcoxy en C₁ à C₄) carbonyle ;
ou Q est le groupe Q₃ 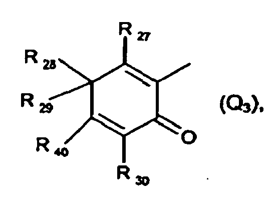 dans lequel
R₂₇ représente un hydroxy ;
R₂₈, R₂₉ et R₄₀, indépendamment l'un de l'autre, représentent un hydrogène ou méthyle ;
R₃₀ représente un méthyle ;
ou Q est le groupe Q₄ 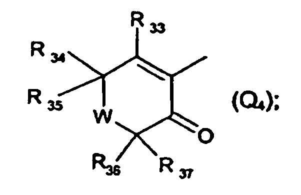 dans lequel
R₃₃ représente un hydroxy ;
W est un oxygène ou C=O ;
R₃₄, R₃₅, R₃₆ et R₃₇, indépendamment l'un de l'autre, représentent un hydrogène ou méthyle, ainsi que les sels agronomiquement acceptables, les isomères et énantiomères de ces composés.

2. Composés selon la revendication 1, dans lesquels Q est Q₁ ou Q₂.

3. Composés selon la revendication 1, dans lesquels L₁ représente le méthylène.

4. Composé selon la revendication 1, dans lequel R₂ représente un alkylsulfonyle en C₁ à C₆.

5. Composition herbicide et d'inhibition de la croissance des plantes, qui comprend une quantité herbicide efficace du composé de formule I sur un support inerte.

6. Procédé de contrôle de la croissance non souhaitée des plantes, qui comprend le traitement des plantes ou du site de celles-ci avec une quantité herbicide efficace d'un composé de formule I ou d'une composition contenant un tel composé.

7. Procédé d'inhibition de la croissance non souhaitée des plantes, qui comprend le traitement des plantes ou de leur site avec une quantité herbicide efficace d'un composé de formule I ou d'une composition contenant un tel composé.

8. Utilisation d'une composition selon la revendication 5 pour contrôler la croissance non souhaitée des plantes.
